# EUROPEAN PATENT APPLICATION

(11) **EP 4 541 906 A1**
(43) Date of publication of application: **23.04.2025**
(21) Application number: 22947071.1
(22) Date of filing: 20.06.2022
(51) Int. Cl.: C12Q 1/686, C12N 15/09, C12Q 1/6813

(54) **PRIMER, DNA DETECTION METHOD, AND DNA DETECTION KIT**

(71) Applicant: HITACHI HIGH-TECH CORPORATION, Tokyo 105-6409 (JP)
(72) Inventor: TANAKA Junko, Tokyo 100-8280 (JP)
(74) Representative: Mewburn Ellis LLP
(86) International application number: PCT/JP2022/024591
(87) International publication number: WO 2023/248310

(57) **Abstract**

The present invention relates to a primer, a DNA detection method, and a DNA detection kit for use in nucleic acid amplification, and particularly in asymmetric nucleic acid amplification. More specifically, this invention relates to a primer for introducing a mutation into a test nucleic acid and amplifying the test nucleic acid, the primer contains, in its sequence, a mutation to be introduced into the test nucleic acid, so as to increase a proportion of single-strand forming bases in a probe binding region of the nucleic acid amplified from the test nucleic acid, at a temperature of the binding of the nucleic acid amplified from the test nucleic acid with the probe; and, a DNA detection method and a DNA detection kit with use of such primer.

## Description

### Technical Field

The present invention relates to a primer, a DNA detection method, and a DNA detection kit for use in nucleic acid amplification, and particularly in asymmetric nucleic acid amplification.

### Background Art

Genetic testing includes techniques such as polymerase chain reaction (PCR), real-time PCR, and digital PCR. The real-time PCR and digital PCR, with a higher accuracy than PCR, use an intercalator or a fluorescent-labeled probe to detect DNA. Hydrolysis probe and molecular beacon are two well-known DNA detection methods with use of the fluorescent-labeled probe. Unlike the hydrolysis probe that liberates a fluorescent dye, upon being degraded, with the aid of nuclease activity of the DNA polymerase, a molecular beacon, which can form a stem-loop in a free state and can bind with a target DNA to be detected at the loop part, is featured by its undegraded nature during PCR, thus enabling not only determination of accomplishment of amplification based on fluorescence intensity, but also melting curve analysis, after PCR.

Regarding DNA detection with use of the molecular beacon aimed at increasing the binding of the molecular beacon with the amplified target DNA, there has been reported a method of asymmetric amplification of target DNA, by unbalancing the concentrations of the forward primer and the reverse primer, so as to excessively amplify a strand complementary to the molecular beacon (NPL 1).

The present inventors have applied the asymmetric nucleic acid amplification with use of the molecular beacon to the digital PCR, and have developed a technique of identifying genotype of the target gene with high sensitivity and high multiplexity, by the post-amplification melting curve analysis (PTL 1 and NPL 2).

An exemplary detection method in the digital PCR will be explained below. First, a DNA polymerase, a primer, and a fluorescent-labeled probe necessary for PCR are added to a limiting diluted sample, to prepare a PCR reaction fluid. The PCR reaction fluid is dispensed into micro partitions such as wells or droplets. Each partition at this time shall either contain one molecule of the target gene, or contain no target gene.

Next, the target gene in the micro partitions is amplified by PCR. After the PCR, the fluorescence intensity in the individual micro partitions is measured. The target gene may be quantified by counting the number of micro partitions that demonstrated fluorescence intensity exceeding a threshold.

In a case where the asymmetric nucleic acid amplification with use of the molecular beacon is applied, implementation of PCR, and the subsequent melting curve analysis of the target gene amplified in the micro partitions and the molecular beacon, can yield the melting temperature (Tm) which is observed as different for each genotype of the target gene, thus enabling genotyping.

### Citation List

### Patent Literature

PTL 1: JP 2018-108063 A

### Non-Patent Literature

NPL 1: BMC Microbiol., 13, pp295, 2013
NPL 2: Anal. Chem., 92, pp11705-11713, 2020

### Summary of Invention

### Technical Problem

The present inventors were, however, the first to recognize that, one strand of the DNA excessively amplified in the asymmetric nucleic acid amplification forms an intramolecular secondary structure, so that the molecular beacon designed in a region having a single-stranded structure can bind to the amplified target DNA, whereas a molecular beacon designed in a region having a double-stranded structure will suffer from lowered proportion of binding, thus causing lowered fluorescence intensity. In particular, in PCR conducted in the micro partitions such as digital PCR, rather than real time PCR conducted in tubes, such lowered fluorescence intensity would make the fluorescence undetectable if dropped below the detection limit, would disable detection of the melting temperature in the melting curve analysis, due to too small change in the fluorescence intensity versus temperature change; or would make the measurement difficult, thus degrading sensitivity and accuracy of the measurement.

It is, therefore, an object of the present invention to provide a novel primer, a DNA detection method, and a DNA detection kit for use in asymmetric nucleic acid amplification, capable of reducing a proportion that a molecular beacon binding region of one strand of the excessively amplified DNA forms a double-stranded structure, and of increasing a proportion that a single stranded structure is formed, thereby suppressing lowering in the proportion of binding between the molecular beacon and amplified DNA, making it possible to detect the target gene to be detected with high accuracy and high sensitivity, and thus enabling genotyping.

### Solution to Problem

The present inventors have revealed that the asymmetric nucleic acid amplification, even with use of the same primer set, can demonstrate the fluorescence intensity largely different depending on the molecular beacon binding region, and that the molecular beacon designed in a region where one amplified chain forms an intramolecular double-stranded structure can yield smaller fluorescence intensity. The present inventors then found that the asymmetric nucleic acid amplification, with use of a primer for use in asymmetric nucleic acid amplification into which a mutation different from the sequence of the target gene is intentionally introduced, successfully lowered the proportion the molecular beacon binding region forms a double-stranded structure, thus increasing the fluorescence intensity. This led the inventors to complete the present invention.

An aspect of this invention relates to a method for detecting a target nucleic acid, the method including:
amplifying a test nucleic acid in the presence of a primer pair that contains a forward primer and a reverse primer, and a probe; and
measuring binding of a nucleic acid amplified by the forward primer and the reverse primer, with the probe,
wherein either the forward primer or the reverse primer, or both contain, in the sequence, a mutation to be introduced into the amplified nucleic acid, so as to increase a proportion of single-strand forming bases in a probe binding region of the amplified nucleic acid, at a temperature of the binding of the amplified nucleic acid with the probe.

Another aspect of this invention relates to a primer for introducing a mutation into a test nucleic acid and amplifying the test nucleic acid, the primer containing, in its sequence, a mutation to be introduced into the test nucleic acid, so as to increase a proportion of single-strand forming bases in a probe binding region of the nucleic acid amplified from the test nucleic acid, at a temperature of the binding of the nucleic acid amplified from the test nucleic acid with the probe.

Another aspect of this invention relates to a kit for detecting a target nucleic acid, the kit including:
a primer pair that contains a forward primer and a reverse primer; and
a probe that binds to a nucleic acid amplified with use of the forward primer and the reverse primer,
wherein either the forward primer or the reverse primer, or both contain, in the sequence, a mutation to be introduced into the amplified nucleic acid, so as to increase a proportion of single-strand forming bases in a probe binding region of the amplified nucleic acid, at a temperature of the binding of the amplified nucleic acid with the probe.

### Advantageous Effects of Invention

This invention can provide a novel primer, a DNA detection method, and a DNA detection kit for use in asymmetric nucleic acid amplification, capable of detecting or quantifying a target gene with higher accuracy and higher sensitivity. This invention is therefore beneficial typically in the fields of basic research, examination, and drug discovery that involve gene detection and genotyping.

### Brief Description of Drawings

[FIG. 1] FIG. 1 contains schematic drawings illustrating an exemplary method for measuring melting temperature of DNA with use of fluorescent-labeled probe.
[FIG. 2] FIG. 2 contains schematic drawings illustrating exemplary behaviors of a fluorescent-labeled probe in symmetric nucleic acid amplification and asymmetric nucleic acid amplification.
[FIG. 3] FIG. 3 contains schematic drawings illustrating binding between a secondary structure of an amplified target DNA and a fluorescent-labeled probe, in asymmetric nucleic acid amplification with use of a mutation-introducing primer, according to one embodiment of this invention.
[FIG. 4] FIG. 4 contains schematic drawings illustrating binding between a secondary structure of an amplified target DNA and a fluorescent-labeled probe, in asymmetric nucleic acid amplification with use of a tagged mutation-introducing primer, according to one embodiment of this invention.
[FIG. 5] FIG. 5 contains drawings illustrating exemplary melting curves observed with use of a mutation-introducing primer, according to one embodiment of this invention.
[FIG. 6] FIG. 6 is a flowchart illustrating an embodiment of a DNA detection method that involves asymmetric nucleic acid amplification with use of a mutation-introducing primer.
[FIG. 7A] FIG. 7A is a drawing illustrating a positional relationship between a secondary structure of an amplified single-stranded DNA to be detected, and a binding region for a fluorescent-labeled probe adapted to wild-type, in Example of this invention.
[FIG. 7B] FIG. 7B is a drawing illustrating a positional relationship between a secondary structure of an amplified single-stranded DNA to be detected, and a binding region for a fluorescent-labeled probe adapted to mutant, in Example of this invention.
[FIG. 7C] FIG. 7C is a drawing illustrating measurement results (differential melting curves) obtained in the absence of the mutation-introducing primer, in Examples of this invention.
[FIG. 8A] FIG. 8A is a drawing illustrating a positional relationship between a secondary structure of an amplified single-stranded DNA to be detected, and a binding region for a fluorescent-labeled probe adapted to wild-type, and a position planned for primer-mediated introduction of mutation.
[FIG. 8B] FIG. 8B is a drawing illustrating a positional relationship between a secondary structure of an amplified single-stranded DNA to be detected, and a binding region for a fluorescent-labeled probe adapted to mutant, and a position of primer-mediated introduction of mutation, in Example of this invention.
[FIG. 9A] FIG. 9A is a drawing illustrating a positional relationship between a secondary structure of an amplified single-stranded DNA to be detected, and a binding region for a fluorescent-labeled probe adapted to wild-type, and a position planned for primer-mediated introduction of mutation.
[FIG. 9B] FIG. 9B is a drawing illustrating a positional relationship between a secondary structure of an amplified single-stranded DNA to be detected, and a binding region for a fluorescent-labeled probe adapted to mutant, and a position of primer-mediated introduction of mutation, in Example of this invention.

### Description of Embodiments

Objects, features, advantages, and ideas thereof of this invention will be apparent to a skilled person in the art from the description of the present specification. A skilled person in the art would easily reproduce the present invention, while referring to the description of this specification. The embodiments, specific examples, and the like of the invention described below illustrate preferred embodiments of the invention, merely for the purpose of exemplification or explanation, without limiting the invention thereto. It will be apparent to a skilled person in the art that various modifications and variations can be made with reference to the description herein, without departing from the spirit and scope of the invention disclosed herein.

### (1) Primer

In one aspect, this invention provides a primer for introducing a mutation into a test nucleic acid and amplifying the test nucleic acid, the primer containing, in its sequence, a mutation to be introduced into the test nucleic acid, so as to increase a proportion of single-strand forming bases in a probe binding region of the nucleic acid amplified from the test nucleic acid, at a temperature of the binding of the nucleic acid amplified from the test nucleic acid with the probe. Such primer may be used as a reagent for introducing a mutation into a test nucleic acid and amplifying the test nucleic acid.

The test nucleic acid in this invention is not particularly limited so long as being a nucleic acid that contains, or possibly contains a target nucleic acid, and may include messenger RNA (mRNA), non-coding RNA (ncRNA), microRNA, genomic DNA, fragments thereof, and hybrid nucleic acids of DNA and RNA, all of which may be single-stranded or double-stranded. If the target nucleic acid is RNA such as mRNA, then the test nucleic acid may be DNA (cDNA, etc.) obtained by reverse transcription of the RNA.

The primer may be a forward primer or a reverse primer, wherein both primers may contain a mutation. The primer may preferably be a reverse primer.

The primer may contain a mutation to be introduced into the test nucleic acid which is then amplified. This mutation may be introduced so as to increase a proportion of single-strand forming bases (proportion of the single-strand forming base to the double-strand forming bases) in a probe binding region of the nucleic acid amplified from the test nucleic acid, at a temperature of the binding of the nucleic acid amplified from the test nucleic acid with the probe. The nucleic acid amplified from the test nucleic acid, although having a single-stranded structure, may occasionally form an internal double-stranded structure, due to presence of complementary bases. What structure the amplified nucleic acid can form depends on its sequence structure, for which a program (such as OligoAnalyzer^{™} Tool (IDT), RNAfold (University of Vienna), etc.) for predicting such structure has been widely used in the art. With use of such program, the structure of the amplified nucleic acid may then be predicted, and the proportion of the single-strand forming bases in the probe binding region of the amplified nucleic acid may be determined. If the proportion of the single-strand forming bases is low, or typically below 50%, a mutation may be introduced into the nucleic acid to be amplified, so as to increase the proportion of the single-strand forming bases at least up to 50%, and preferably up to 60% or above.

The primer may be designed by a conventional primer design method, except for containing therein the aforementioned mutation, so as to have a length, sequence composition, and melting temperature suitable for causing specific binding to the target nucleic acid.

The primer may further contain a tag sequence, for the purpose of increasing the proportion of the single-strand forming bases. Such tag sequence may be designed to have a length and composition not affective to detection of the target nucleic acid. The tag sequence may be contained in either of the forward primer or the reverse primer, or in both primers. The nucleic acid thus amplified with use of such primer will contain the tag sequence.

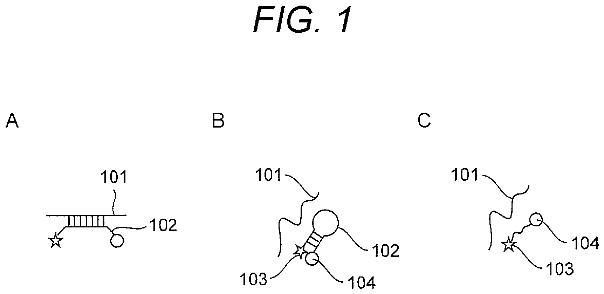

One embodiment disclosed herein relates to a primer for use in asymmetric nucleic acid amplification, having a mutation introduced therein, so that the fluorescent-labeled probe binding region of the target DNA can reduce the proportion of the double-stranded structure, and can increase the proportion of the single-stranded structure, at a temperature at which the fluorescent-labeled probe such as molecular beacon can bind. The primer will be specifically described with reference to the schematic drawings in FIGs. 1 to 5.

FIG. 1 is a schematic diagram illustrating a behavior of a fluorescent-labeled probe, under varied temperature of a reaction fluid. FIG. 1 will be described with reference to a molecular beacon, although the fluorescent-labeled probe may be exemplified by molecular beacon and Taqman probe.

The molecular beacon may be designed to be an oligonucleotide, having a sequence complementary to a sequence between the paired primers used in nucleic acid amplification for amplifying a target gene. The molecular beacon also has complementary sequences at both ends thereof, with a fluorescent dye 103 provided to one end, and a quenching dye (quencher) 104 provided to the other end. In an initial state of nucleic acid amplification, the molecular beacon 102 may be freed alone as illustrated in FIG. 1, B. The molecular beacon 102 in this state may form a stem-loop, with the fluorescent dye 103 and the quencher 104 arranged in proximity, thus causing no fluorescence emission. Upon heating of a test fluid in the first denaturation step, the molecular beacon can exist with a high degree of freedom as illustrated in FIG. 1, C, but still remains quenched since the fluorescent dye and the quenching dye are constantly kept in proximity. Upon cooling down in the annealing step to reach a temperature at or around room temperature, the loop part of the molecular beacon 102 may anneal to the DNA 101 amplified in the test fluid, as illustrated in FIG. 1, A. This allows the fluorescent-labeled probe 102 to emit strong fluorescence, since the fluorescent dye 103 and the quencher 104 may be kept apart. In the next extension step, the molecular beacon 102 may be released, again to have a quenched form as illustrated in FIG. 1, B. In the next denaturation step, the molecular beacon will be again in the state illustrated in FIG. 1, C, while kept quenched. Since nucleic acid amplification proceeds while repeating these steps, so that the fluorescence intensity may only be measured at any stage, during heating or cooling. The fluorescence intensity can be measured in the same manner, also after completion of the nucleic acid amplification. The fluorescence intensity may even be measured after completion of the nucleic acid amplification, upon heating or cooling conducted solely for the purpose of the measurement.

When using a DNA intercalator, in place of combination of the fluorescent dye and the quencher, the DNA intercalator may intercalate in the double strand of the test DNA during its double-stranded stage to emit fluorescence, meanwhile may exit the test DNA during its single-stranded stage to quench the fluorescence. The fluorescence intensity may only be measured again similarly to the molecular beacon 102, at any stage of the nucleic acid amplification, during heating or cooling. The fluorescence intensity may even be measured after completion of the nucleic acid amplification, upon heating or cooling conducted solely for the purpose of the measurement.

In the molecular beacon 102 used herein, the combination of the fluorescent dye 103 and the quencher 104 is not particularly limited, as long as it is a combination widely adopted to real-time PCR. The fluorescent dye 103 may include FAM, VIC, ROX, Cy3 and Cy5; meanwhile the quencher 104 may include TAMRA, BHQ1, BHQ2 and BHQ3. All of them have widely been used, and are commercially available.

When using two types of target gene having different sequences, these two types of target gene may be separately detected in a single reaction system, with use of molecular beacons 102 having sequences specifically capable of binding to the individual target genes, with different fluorescent dyes bound thereto.

The DNA intercalator is not particularly limited as long as it can increase the fluorescence intensity upon binding with the double-stranded DNA, and is usable for detection of the double-stranded DNA. The DNA intercalator applicable herein may specifically include SYBR (registered trademark) Green I, SYBR Gold, PicoGreen (registered trademark), SYTO (registered trademark) Blue, SYTO Green, SYTO Orange, SYTO Red, POPO (registered trademark)-1, BOBO (registered trademark)-1, YOYO (registered trademark)-1, TOTO (registered trademark)-1, JOJO (registered trademark)-1, POPO-3, LOLO (registered trademark)-1, BOBO-3, YOYO-3, TOTO-3, PO-Pro (registered trademark)-1, YO-Pro (registered trademark)-1, TO-Pro (registered trademark)-1, JO-Pro (registered trademark)-1, PO-Pro-3, YO-Pro-3, TO-Pro-3, TO-Pro-5, and ethidium bromide, all of them commercially available. The DNA intercalator, if being heat resistant, may be added to the reaction fluid in advance of the nucleic acid amplification.

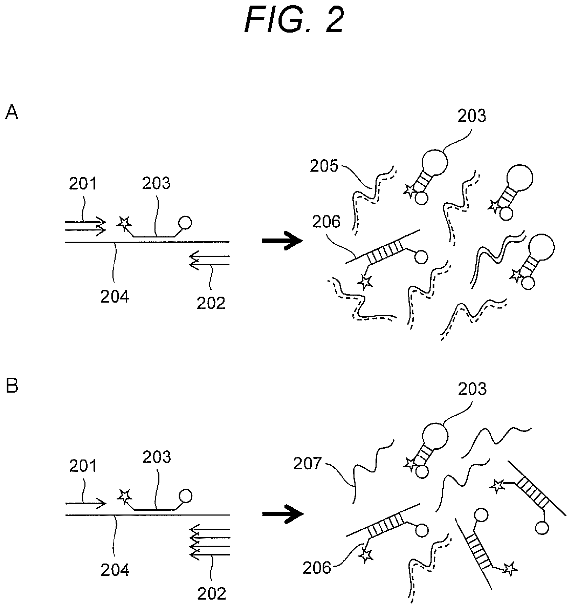

FIG. 2 contains schematic drawings illustrating exemplary behaviors of a fluorescent-labeled probe in symmetric nucleic acid amplification and asymmetric nucleic acid amplification. In a case where the forward primer 201 and the reverse primer 202 are present at the same concentration as illustrated in FIG. 2, A, both of the complementary strands of the DNA 204 to be detected may be amplified in equal amounts, thus yielding a large amount of a molecule 205 having therein these complementary chains in the form of double strand, meanwhile a very small amount of a molecule 206 having therein the fluorescent-labeled probe 203 bound to a single-strand forming molecule may be produced. In a case where the forward primer 201 and the reverse primer 202 are present at asymmetric concentrations as illustrated in FIG. 2, B (the reverse primer 202 is increased in FIG. 2, B), one strand 207 of the DNA 204 to be detected, which is a complementary strand of the molecular beacon, may excessively be amplified, thus yielding a large amount of the molecule 206 having therein the fluorescent-labeled probe 203 bound to the single-strand forming molecule. The asymmetric nucleic acid amplification thus conducted as above can increase the amount of binding of the fluorescent-labeled probe with the amplified DNA to be detected, thereby enhancing the fluorescence intensity.

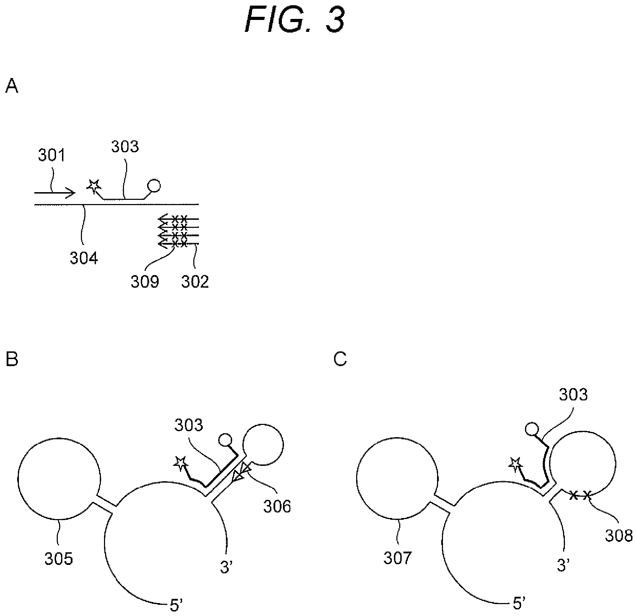

FIG. 3 contains schematic drawings illustrating binding between a secondary structure of an amplified target DNA and a fluorescent-labeled probe, in asymmetric nucleic acid amplification with use of a mutation-introducing primer. As illustrated in FIG. 3, A, a forward primer 301, a mutation-introducing reverse primer 302 having a mutation 309 introduced therein, and a fluorescent-labeled probe 303 may be prepared for a target DNA 304. The concentration of the reverse primer 302 may be set excessive over the concentration of the forward primer 301, thus allowing asymmetric nucleic acid amplification to proceed. FIG. 3, B is a schematic drawing illustrating binding between a secondary structure 305 of an amplified single-stranded target DNA and a fluorescent-labeled probe 303, in asymmetric nucleic acid amplification with use of a reverse primer having no mutation 309 introduced therein (conventional method). Since the mutation 309 is not introduced, a position 306 on the secondary structure 305 of the amplified DNA remains unchanged from the original base of the target DNA. In the secondary structure 305 of the amplified single-stranded target DNA, a binding region for the fluorescent-labeled probe 303 may form a double-stranded structure, thus reducing the amount of binding of the fluorescent-labeled probe 303. Meanwhile, FIG. 3, C is a schematic drawing illustrating binding between a secondary structure 307 of an amplified single-stranded target DNA and a fluorescent-labeled probe 303, in asymmetric nucleic acid amplification with use of the mutation-introducing reverse primer 302 (this invention). With the mutation 308 introduced while mediated by the mutation-introducing reverse primer 302, the amplified single-stranded target DNA will have a secondary structure 307, which is different from the secondary structure 305 of the amplified single-stranded target DNA illustrated in FIG. 3, B. Hence, the binding region for the fluorescent-labeled probe 303 in FIG. 3, C will become less likely to form the double-stranded structure, but will become more likely to form the single-stranded structure as compared with that in FIG. 3, B, thus increasing the amount of binding of the fluorescent-labeled probe 303.

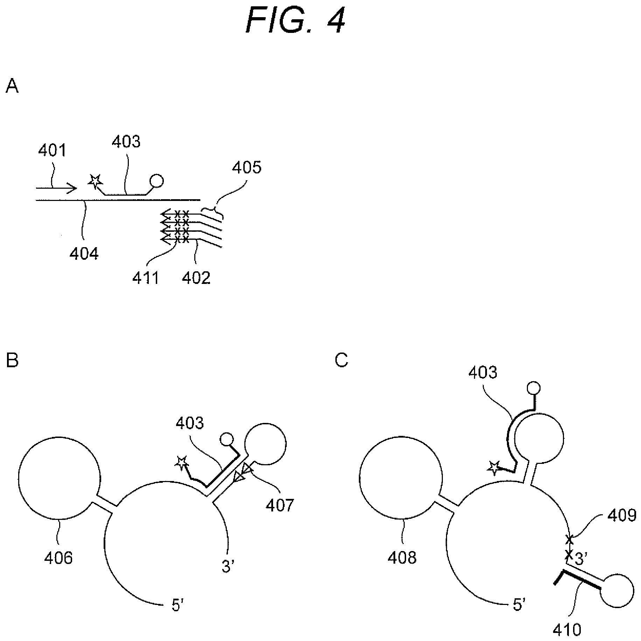

FIG. 4 contains schematic drawings illustrating binding between a secondary structure of an amplified target DNA and a fluorescent-labeled probe, in asymmetric nucleic acid amplification with use of a tagged mutation-introducing primer. As illustrated in FIG. 4, A, a forward primer 401, a mutation-introducing reverse primer 402 having a tag sequence 405 added thereto and a mutation 411 introduced therein, and a fluorescent-labeled probe 403 may be prepared for a target DNA 404. The concentration of the reverse primer 402 may be set excessive over the concentration of the forward primer 401, thus allowing asymmetric nucleic acid amplification to proceed. FIG. 4, B is a schematic drawing illustrating binding between a secondary structure 406 of an amplified single-stranded target DNA and a fluorescent-labeled probe 403, in asymmetric nucleic acid amplification with use of a reverse primer (not illustrated) having neither tag sequence nor mutation 411 introduced therein (same as FIG. 3, B). Since the mutation 411 is not introduced, a position 407 on the secondary structure 406 of the amplified DNA may remain unchanged from the original bases of the target DNA. In the secondary structure 406 of the amplified single-stranded target DNA, a binding region for the fluorescent-labeled probe 403 may form a double-stranded structure, thus reducing the amount of binding of the fluorescent-labeled probe 403. Meanwhile, FIG. 4, C is a schematic drawing illustrating binding between a secondary structure 408 of an amplified single-stranded target DNA and a fluorescent-labeled probe 403, in asymmetric nucleic acid amplification with use of the tagged mutation-introducing reverse primer 402. With a mutation 409 and a tag sequence 410 introduced while mediated by the tagged mutation-introducing reverse primer 402, the amplified single-stranded target DNA will have a secondary structure 408, which is different from the secondary structure 406 of the amplified single-stranded target DNA illustrated in FIG. 4, B. Hence, the binding region for the fluorescent-labeled probe 403 in FIG. 4, C will become less likely to form the double-stranded structure, but will become more likely to form the single-stranded structure as compared with that in FIG. 4, B, thus increasing the amount of binding of the fluorescent-labeled probe 403.

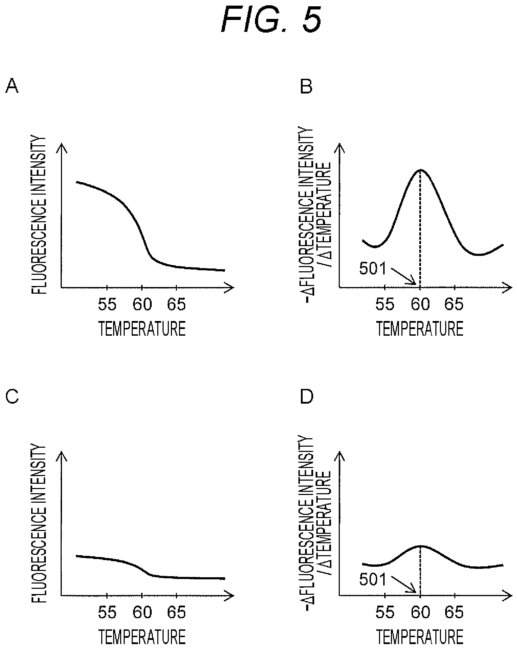

FIG. 5 illustrates exemplary melting curves observed in asymmetric nucleic acid amplification with use of a mutation-introducing primer, and in asymmetric nucleic acid amplification without use of a mutation-introducing primer. In the asymmetric nucleic acid amplification with use of the mutation-introducing primer, measurement of temperature-dependent changes in the fluorescence intensity of the fluid may give a melting curve illustrated in FIG. 5, A, demonstrating a large change in the fluorescence intensity. A differential curve derived therefrom, illustrated in FIG. 5, B, demonstrates a large peak, wherein temperature at the peak indicates the melting temperature (Tm) 501. On the other hand, in the asymmetric nucleic acid amplification without use of the mutation-introducing primer, measurement of temperature-dependent changes in the fluorescence intensity of the solution demonstrates a fluorescence intensity illustrated in FIG. 5, C, which is lowered from that in FIG. 5, A. Hence, a differential curve derived therefrom demonstrates a small peak as illustrated in FIG. 5, D. The peak, having a broad shape, would indicate an erroneous value, which is different from the melting temperature of the target gene in the fluid. With an even greater degree of decrease in the fluorescence intensity, the fluorescence intensity resulted from the amplification of the target gene will fall under the detection limit, and will become undetectable.

### (2) DNA Detection Method

In another aspect, this invention relates to a method for detecting a target nucleic acid, the method including:
amplifying a test nucleic acid in the presence of a primer pair that includes a forward primer and a reverse primer, and a probe; and
measuring binding of a nucleic acid amplified by the forward primer and the reverse primer, with the probe,
wherein either the forward primer or the reverse primer, or both contain, in the sequence, a mutation to be introduced into the amplified nucleic acid, so as to increase a proportion of single-strand forming bases in a probe binding region of the amplified nucleic acid, at a temperature of the binding of the amplified nucleic acid with the probe.

Detection of the target nucleic acid described herein typically means checking the presence or absence of the target nucleic acid, measuring the concentration or abundance ratio of the target nucleic acid, identifying the target nucleic acid, and discriminating the genotype.

Either the forward primer or the reverse primer, or both are the primers described in the previous section.

The probe may contain a fluorescent dye, or may contain a fluorescent dye and a quenching dye. In a case where the probe contains a fluorescent dye, the binding between the amplified nucleic acid and the probe may be measured with use of the fluorescent dye. In one embodiment, a 3' end sequence and a 5' end sequence of the probe may be complementary. When the probe remains unbound to the amplified nucleic acid, the 3' end sequence and the 5' end sequence may bind to form a stem structure. In a case where the probe does not contain the fluorescent dye, a DNA intercalator for example may be used.

The amplifying step may be conducted by any amplification reaction which is amplification reactions known in the art. The amplifying step may preferably be conducted by asymmetric nucleic acid amplification. The amplification reaction in this case is allowed to proceed, with an increased amount of addition of either the forward primer or the reverse primer (for example, a mutation-containing primer). In a preferred embodiment, the amplification reaction may be allowed to proceed, with the amount of addition of a mutation-containing reverse primer increased over the forward primer. See the description of Anal. Chem., 92, pp11705-11713, 2020 (NPL 2) for the asymmetric nucleic acid amplification.

In one embodiment, the binding of the amplified nucleic acid with the probe may be measured with changing temperature. Since the binding changes depending on the melting temperature, the melting temperature of the double strand of the amplified nucleic acid and the probe may be estimated, with reference to such change in the binding due to the temperature change.

In one embodiment, binding of the amplified nucleic acid with the probe may be measured for each cycle of temperature change. The target nucleic acid may be detected from a relationship between the number of cycles and a change in the binding.

With use of a plurality of types of the mutation-containing primer, a plurality of target nucleic acids may be detected. In this case, binding of the amplified nucleic acids with the probe may be measured for each cycle of temperature change. Ratio of the initial concentrations of the plurality of target nucleic acids in the test nucleic acid may be estimated, from a relationship between the number of cycles and changes in the binding.

In one embodiment, binding of the amplified nucleic acid with the probe may be measured for each cycle of temperature change. An initial concentration of the target nucleic acid whose concentration is unknown in the test nucleic acid may be estimated by comparing a relationship between the number of cycles and a change in the binding, with a change in the binding of a sample that contains a known concentration of the target nucleic acid.

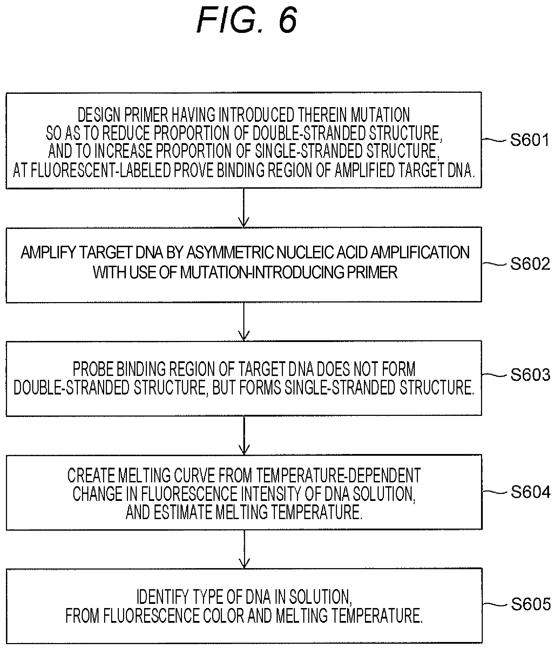

The DNA detection method disclosed herein may include:
subjecting a solution that contains a target DNA to asymmetric nucleic acid amplification with use of:
a primer that introduces a mutation, so that a fluorescent-labeled probe binding region of the target DNA can reduce a proportion of a double-stranded structure, and can increase a proportion of a single-stranded structure, at a temperature of the binding of the fluorescent-labeled probe,
a fluorescent probe that binds to the target DNA,
a primer that is paired with the mutation-introducing primer, for amplifying the fluorescent probe binding region of the target DNA, and
a DNA polymerase that amplifies the target DNA;
measuring change in fluorescence intensity of a DNA solution with changing temperature; and
estimating a melting temperature of the DNA double strand from the change in fluorescence intensity of the DNA solution due to the temperature change. The DNA detection method will be specifically described with reference to the schematic drawing in FIG. 6.

First, designed is a primer having introduced therein a mutation so as to reduce a proportion of a double-stranded structure, and to increase a proportion of a single-stranded structure, at the fluorescent-labeled probe binding region of the amplified target DNA (S601). The mutation-introducing primer herein may have a tag sequence, but not necessarily. Next, the target DNA may be amplified by the asymmetric nucleic acid amplification with use of the mutation-introducing primer (S602). The target DNA after the asymmetric nucleic acid amplification may form a secondary structure in which the probe binding region becomes less likely to form the double-stranded structure, and may become more likely to form a single-stranded structure (S603). Temperature-dependent change in fluorescence intensity of the solution of the target DNA after the asymmetric nucleic acid amplification may be measured, a melting curve may be created, and the melting temperature may be estimated from a peak temperature appeared in a differential curve of the melting curve (S604). Lastly, the type of the target DNA contained in the solution may be identified, from the fluorescence color of the solution of the target DNA, and the melting temperature (S605).

If there are a plurality of types of target DNA, a plurality of primers and a plurality of probes may be prepared in accordance with the sequence to be detected, added at the same time, and the DNA solution that contains the plurality of the target DNAs may be subjected to the asymmetric nucleic acid amplification. With the plurality of probes designed while changing the melting temperature with respect to the individual target DNAs, or changing the type of fluorescent dye, it now becomes possible to identify, after the asymmetric nucleic acid amplification, the types of the target DNA contained in the solution, from the fluorescence color of the solution and the melting temperature. This is exemplified by a case where the target gene contains a plurality of types of wild-type alleles and mutant alleles, wherein the plurality of types of target gene are not particularly limited thereto.

A process of the asymmetric nucleic acid amplification with use of the mutation-introducing primer may also be monitored in real time, with use of a fluorescent-labeled probe. For example, a proportion of the initial concentrations of the target DNA, contained in the plurality of solutions, may be estimated in the asymmetric nucleic acid amplification, by going through the step of measuring the fluorescence intensity for each cycle of temperature change, and from the relationship between the number of cycles and changes in the fluorescence intensity. Moreover, it is also possible herein to estimate the initial concentration of the target DNA in the sample whose concentration is unknown, by going through a step of preparing, as a control, a known concentration of the target DNA, and of measuring the control at the same time, to obtain the fluorescence intensity for each cycle of temperature change, and by comparing the relationship between the number of cycles and changes in the fluorescence intensity, with changes in the fluorescence intensity of the sample of a known concentration.

The asymmetric nucleic acid amplification with use of the mutation-introducing primer may also be applicable to digital PCR. Alternatively, the reaction fluid that contains the mutation-introducing primer may be dispensed into micro partitions, amplified, and then subjected to the melting curve analysis, thereby detecting the target gene contained in the micro partitions (genotyping, for example).

In an exemplary case where the DNA solution to be tested contains a target gene P and a target gene Q, the micro partitions that contain the target gene P will cause therein hybridization of a fluorescent-labeled probe adopted to the target gene P, with the DNA amplified by PCR, and will emit fluorescence. By analyzing the thus emitted fluorescence, the melting temperature of the target gene P and the adapted fluorescent-labeled probe may be estimated. On the other hand, the micro partitions that contain the target gene Q will cause therein hybridization of a fluorescent-labeled probe adopted to the target gene Q, with the DNA amplified by PCR, and will emit fluorescence. By analyzing the thus emitted fluorescence, the melting temperature of the target gene Q and the adapted fluorescent-labeled probe may be estimated. In this way, presence or absence of the target gene P, and presence or absence of the target gene Q, may be determined, with reference to the fluorescence intensity, the type of fluorescence (color, for example), and the melting temperature.

Since the melting temperature of DNA is not affected by PCR reaction efficiency, in-plane measurement variation during the fluorescence measurement, and the like, so that use of the melting temperature of DNA enables highly accurate determination of the types of DNA (genotype, for example) in the micro partitions. For example, by preliminarily determining the sequences of the fluorescent-labeled probes so that the individual fluorescent-labeled probes will demonstrate different melting temperatures (Tm) with respect to the target genes, by measuring the temperature-dependent changes in the fluorescence intensity, by analyzing the melting curve, and by comparing the melting temperature, it now becomes possible to detect DNAs in the micro partitions (genotyping, for example).

In the simultaneous detection of the plurality of types of target gene, the distinction of them may be made with reference to a reference temperature and the measured melting temperature, and preferably with reference to a reference temperature range. For example, if the measured melting temperature obtained from a certain well was found to fall in a predetermined range (for example, within the reference melting temperature ±1°C) that covers the reference melting temperature recorded in the database regarding a certain target gene, such well is judged to contain the target gene. Use of such range of reference melting temperature enables more accurate determination, while giving proper consideration to an allowable range.

Alternatively, a proportion or a difference of the fluorescence intensities at different temperatures may be used as the information regarding the fluorescence intensity. The fluorescence intensity can be standardized, for example, by using a proportion or a difference between the fluorescence intensity at a temperature lower than the reference melting temperature, and the fluorescence intensity at a temperature higher than the reference melting temperature. For example, if the proportion or the difference for a certain well falls in a predetermined range, the well is then judged to be positive, whereas in not, the well is judged to be negative.

Influence of the fluorescent-labeled probe *per se,* that is, influence of the background influence, may be eliminated, typically by subtracting the fluorescence intensity at 85°C from the fluorescence intensity at 50°C.

A method for determining the range of the fluorescence intensity, or the range of the reference melting temperature may be freely selected. For example, the range may be statistically determined by an operator from a result of pilot experiment or the like conducted in advance, or may be automatically determined by a DNA detection system. Alternatively, a threshold of the fluorescence intensity and a predetermined range of the reference melting temperature may be statistically determined, with use of measurement data from the individual wells in a cartridge, measured every time the digital PCR takes place.

The data, from which DNA in the wells is statistically determined, may contain any of, or all of the items below, or even any item other than these items.
- Fluorescence intensity at temperature lower than reference melting temperature
- Fluorescence intensity at temperature higher than reference melting temperature
- Proportion of fluorescence intensity at temperature lower than reference melting temperature, to fluorescence intensity at temperature higher than reference melting temperature
- Difference between fluorescence intensity at temperature lower than reference melting temperature, and fluorescence intensity at temperature higher than reference melting temperature
- Feature that represents reference melting temperature
- Feature that represents shape of melting curve

With the secondary structure of the thus-amplified nucleic acid changed so as to increase a proportion of single-stranded forming base in the probe binding region, the amount of binding to the probe binding region in the amplified nucleic acid increases, thereby improving the measurement sensitivity and the measurement accuracy, such as genotyping accuracy.

### (3) DNA Detection Kit

The aforementioned method of this invention can be implemented more easily and simply, by using a kit that contains at least a primer for introducing a mutation. That is, in one aspect, this invention relates to a kit for detecting a target nucleic acid, the kit including:
a primer pair that contains a forward primer and a reverse primer; and
a probe that binds to a nucleic acid amplified with use of the forward primer and the reverse primer,
wherein either the forward primer or the reverse primer, or both contain, in the sequence, a mutation to be introduced into the amplified nucleic acid, so as to increase a proportion of single-strand forming bases in a probe binding region of the amplified nucleic acid, at a temperature of the binding of the amplified nucleic acid with the probe.

The forward primer and the reverse primer are as described in the preceding section. In one embodiment, the concentration of the forward primer and the concentration of the reverse primer contained in the kit of this invention may be different, and the concentration of either one (preferably, a primer for introducing a mutation) may be set higher.

In one embodiment, the kit of this invention may contain a plurality of types of the primer pair containing the forward primer and the reverse primer, individually adapted to a plurality of target nucleic acids. In one embodiment, the kit of this invention may contain a plurality of types of the probe individually adapted to a plurality of target nucleic acids.

The kit of this invention may further contain any other component necessary for implementing the amplification reaction, such as DNA polymerase or substrate. The kit may also contain a package insert describing a procedure or a protocol for detecting the target nucleic acid.

In one embodiment, the DNA detection kit disclosed herein may contain: a first primer, at a first concentration, that introduces a mutation so that a fluorescent-labeled probe binding region of the target DNA can reduce a proportion of a double-stranded structure, and can increase a proportion of a single-stranded structure, at a temperature of the binding of the fluorescent-labeled probe; a fluorescent probe that binds to the target DNA;
a second primer, at a concentration different from the first concentration, that is paired with the mutation-introducing primer, for amplifying the fluorescent probe binding region of the target DNA; and a DNA polymerase that amplifies the target DNA.

The first primer for introducing a mutation, and the second primer paired therewith for use in the asymmetric nucleic acid amplification may have different concentrations, wherein either the first primer for introducing a mutation, or the second primer may have higher concentration. The first primer for introducing a mutation may contain not only the mutation, but also a tag sequence.

If there are a plurality of target DNAs, a plurality of primers and a plurality of probes may be prepared in accordance with the sequence to be detected, added at the same time, and the DNA solution that contains the plurality of the target DNAs may be subjected to the asymmetric nucleic acid amplification. With the plurality of probes designed while changing the melting temperature with respect to the individual target DNAs, or changing the type of fluorescent dye, it now becomes possible to identify, after the asymmetric nucleic acid amplification, the types of the target DNA contained in the solution, from the fluorescence color of the solution and the melting temperature.

### (4) Primer Design Method

This invention also provides a method for designing a primer for use in detection of a target nucleic acid, on the basis of amplification of a test nucleic acid, and measurement of binding of the amplified product with a probe. Such method may include: designing a primer pair for amplifying a test nucleic acid; determining a proportion of single-strand forming bases in a probe binding region of the nucleic acid amplified by the designed primer pair; and introducing a mutation into one or both primers of the designed primer pair, so that a proportion of the single-strand forming bases will be at least 50%. The method may further include determining the proportion of the single-stranded forming bases in the probe binding region of the nucleic acid amplified by the primer pair that contains the primer having a mutation introduced therein; and checking whether or not the proportion of the single-stranded forming bases is at least 50%.

### Examples

### [Example 1]

This Example will explain an exemplary asymmetric nucleic acid amplification with use of a mutation-introducing primer.

First, asymmetric nucleic acid amplification without use of a mutation-introducing primer, and subsequent melting curve analysis will be described. A genomic DNA of a G13D mutant of KRAS gene (final concentration: 133 molecules/µL) was prepared, to which a forward primer (final concentration: 0.25 µM), a reverse primer (final concentration: 2.0 µM), a fluorescent-labeled probe adapted to wild-type (final concentration: 0.5 µM), a fluorescent-labeled probe adapted to G13D mutant (final concentration: 0.5 µM), and 1x Master Mix (containing DNA polymerase and dNTP), all necessary for PCR, were added. A PCR reaction fluid was thus prepared. The primer pair herein was added at an asymmetric concentration, so that the complementary DNA strand of the fluorescent-labeled probe would be excessively amplified. Sequences of the primers and the probes are as follows. Note that all of the fluorescent-labeled probes have, located near both ends thereof, complementary sequences that can form an intramolecular double strand. The fluorescent-labeled probe adapted to wild-type has HEX as a fluorescent dye bound to the 5' end, and has BHQ-1 as a quencher bound to the 3' end. Meanwhile, the fluorescent-labeled probe adapted to mutant has FAM as a fluorescent dye bound to the 5' end, and has BHQ-1 as a quencher bound to the 3' end.
Forward primer: 5'-GTCACATTTTCATTATTTTTATTATAAGG-3' (SEQ ID NO: 1)
Reverse primer: 5'-GTATCGTCAAGGCACTCTTGCC-3' (SEQ ID NO: 2)
Fluorescent-labeled probe adapted to wild-type: 5'-TTGGAGCTGGTGGCGT-3' (SEQ ID NO: 3)
Fluorescent-labeled probe adapted to mutant: 5'-CTGGTGACGTAGGCA-3' (SEQ ID NO: 4)

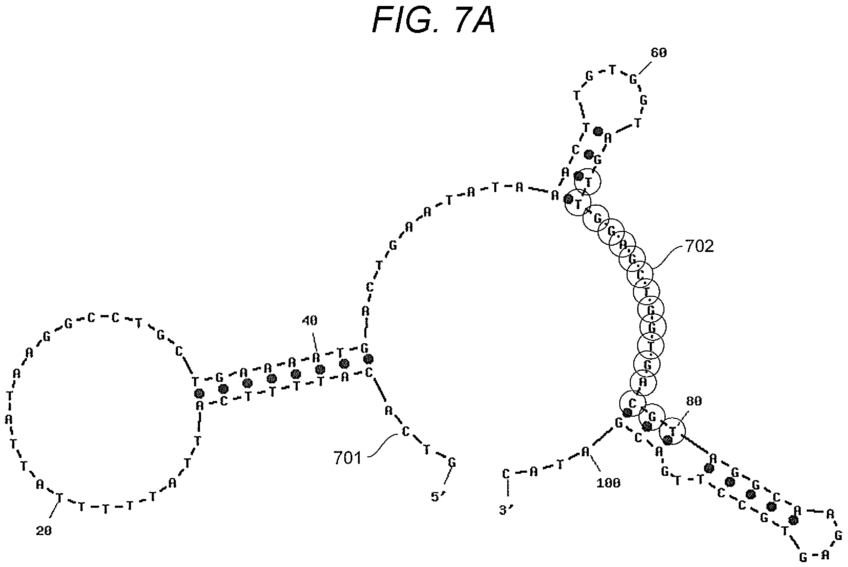

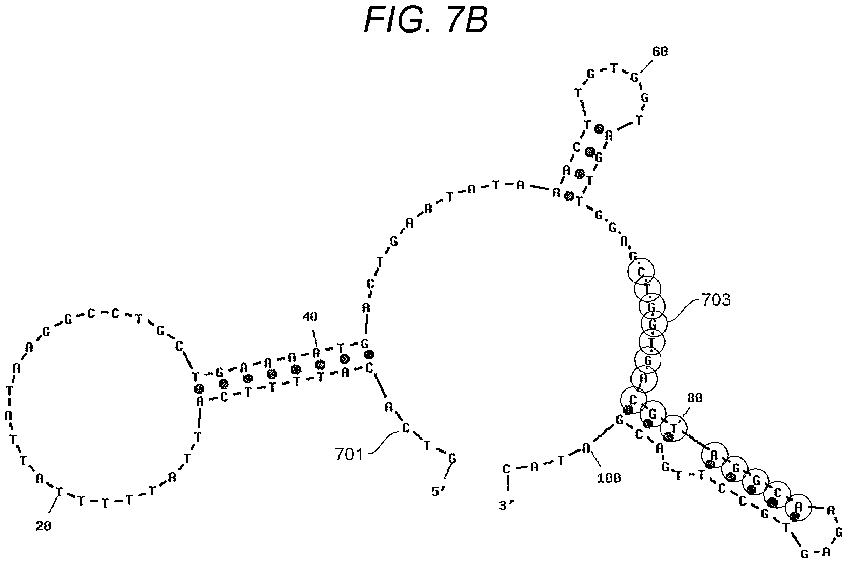

FIG. 7A illustrates a positional relationship between a secondary structure 701 of an amplified single-stranded G13D mutant DNA (SEQ ID NO: 5), and a binding region 702 for a fluorescent-labeled probe adapted to wild-type. FIG. 7B illustrates a positional relationship between a secondary structure 701 of an amplified single-stranded G13D mutant DNA (SEQ ID NO: 5), and a binding region 703 for a fluorescent-labeled probe adapted to mutant. The binding region 702 for the fluorescent-labeled probe in FIG. 7A demonstrates a single-strand forming rate of 69%, meanwhile the binding region 703 for the fluorescently labeled probe in FIG. 7B demonstrates a single-strand forming rate of 47%.

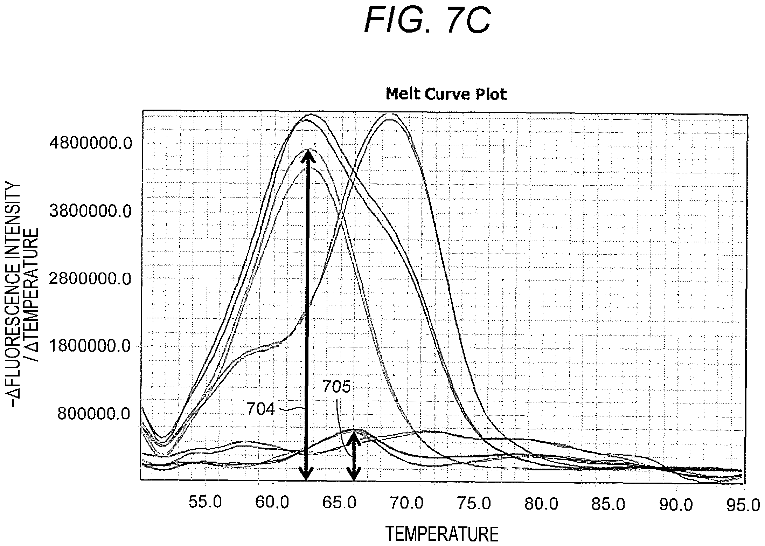

FIG. 7C illustrates differential curves of the melting curves, resulted from the melting curve analysis after the asymmetric nucleic acid amplification. As compared with a peak 704 that appeared in the differential curve of the melting curve of the amplified G13D mutant DNA 701 and the fluorescent-labeled probe 702 adapted to wild-type, a peak 705 that appeared in the differential curve of the melting curve of the amplified G13D mutant DNA 701 and the fluorescent-labeled probe 703 adapted to mutant, was found to be smaller. The result revealed that a low rate of single-strand formation of the binding region for the fluorescent-labeled probe will reduce the amount of binding between the amplified DNA and the fluorescent-labeled probe. This would further reduce the amount of fluorescence and would make the detection difficult, since the mutant might exist only in a small amount.

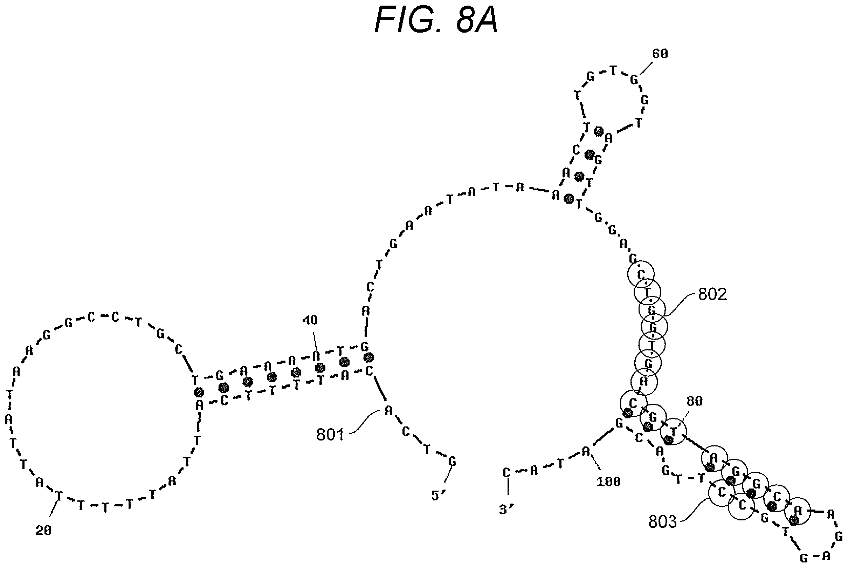

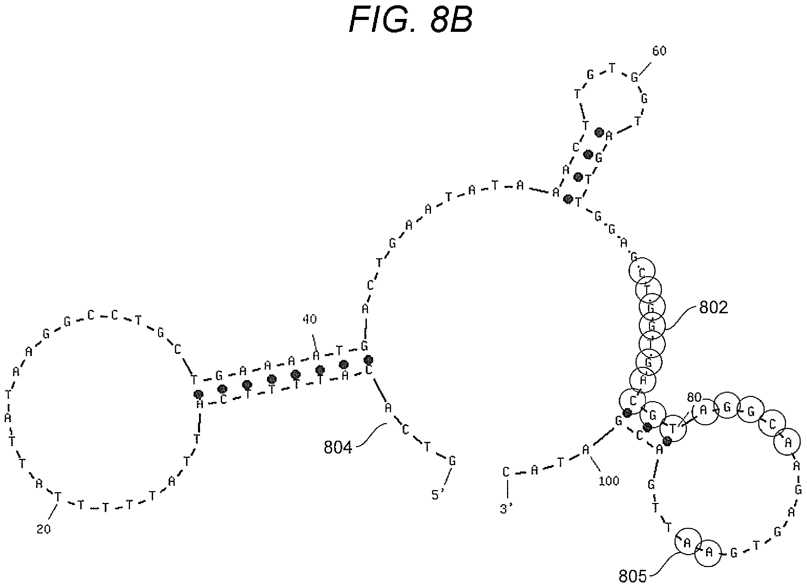

Next, results of the asymmetric nucleic acid amplification with use of a mutation-introducing primer will be illustrated in FIGs. 8A and 8B. FIG. 8A illustrates a position 803 planned for primer-mediated introduction of a mutation, in the positional relationship between a secondary structure 801 of the amplified single-stranded G13D mutant DNA (SEQ ID NO: 5), and a binding region 802 for the fluorescent-labeled probe adapted to mutant, in a case where the mutation-introducing primer illustrated in FIG. 7A was not used. Then, a mutation-introducing primer, in which a base sequence CC at the position 803 planned for primer-mediated introduction of mutation is replaced with a base sequence AA, was designed, and used for the asymmetric nucleic acid amplification. FIG. 8B illustrates a positional relationship between a secondary structure 804 of the thus amplified single-stranded G13D mutant DNA (SEQ ID NO: 6), and the binding region 802 for the fluorescent-labeled probe adapted to mutant, in which a position 805 where the mutation was introduced by the primer was additionally illustrated. As illustrated in FIG. 8B, the binding region 802 for the fluorescent-labeled probe adopted to the mutant will become more likely to form the single strand, thus increasing the amount of binding of the fluorescent-labeled probe.

The asymmetric nucleic acid amplification, thus conducted with use of the mutation-introducing primer, can change the secondary structure of the target gene to be amplified so as to increase the proportion of single-strand formation, thereby increasing the amount of binding of the fluorescent-labeled probe, and improving sensitivity and accuracy of the measurement.

### [Example 2]

This Example will explain an exemplary asymmetric nucleic acid amplification with use of a tagged mutation-introducing primer.

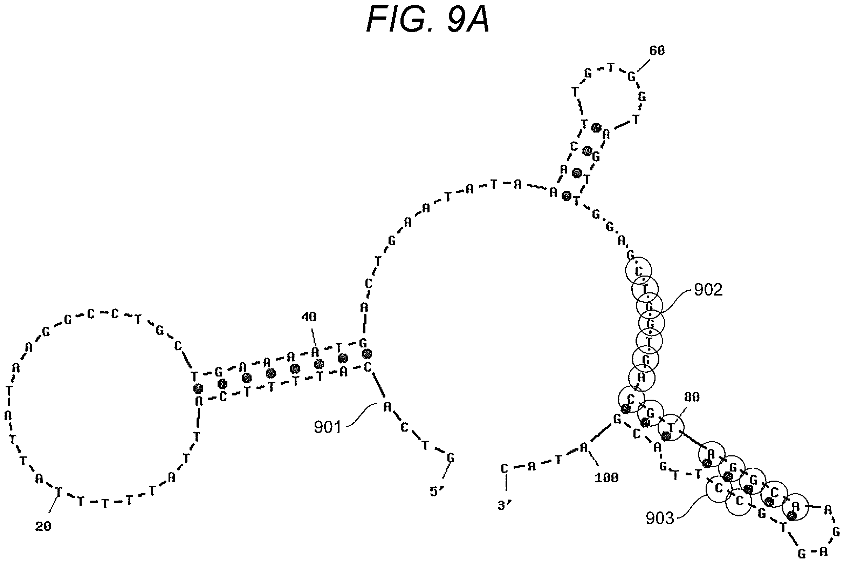

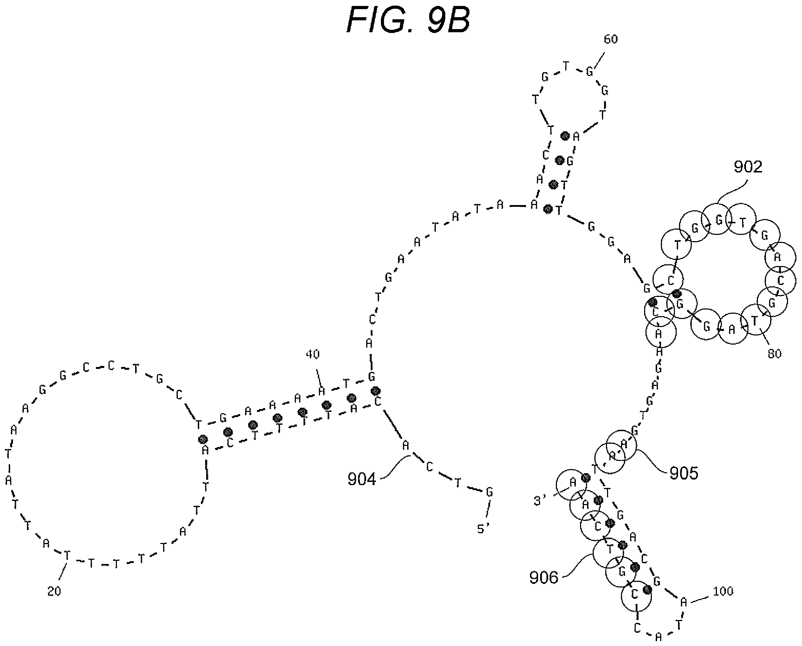

Results of the asymmetric nucleic acid amplification with use of a tagged mutation-introducing primer will be illustrated in FIGs. 9A and 9B. FIG. 9A illustrates a position 903 planned for primer-mediated introduction of a mutation, in the positional relationship between a secondary structure 901 of the amplified single-stranded G13D mutant DNA (SEQ ID NO: 5), and a binding region 902 for the fluorescent-labeled probe adapted to mutant, in a case where the mutation-introducing primer illustrated in FIG. 7A was not used. A mutation-introducing primer, in which a base sequence CC at the position 903 planned for primer-mediated introduction of mutation is replaced with a base sequence AA, and a tag sequence is added to the 3' end, was designed, and used for the asymmetric nucleic acid amplification. FIG. 9B illustrates a positional relationship between a secondary structure 904 of the thus amplified single-stranded G13D mutant DNA (SEQ ID NO: 7), and the binding region 902 for the fluorescent-labeled probe adapted to mutant, in which a position 905 where the mutation was introduced by the primer, and a position 906 where the tag was added by the primer, were additionally illustrated. As illustrated in FIG. 9B, the binding region 902 for the fluorescent-labeled probe adopted to the mutant will become more likely to form the single strand, thus increasing the amount of binding of the fluorescent-labeled probe. Note as illustrated in FIGs. 9A and 9B, that the secondary structure of the single-stranded DNA may greatly change before and after the introduction of mutation.

The asymmetric nucleic acid amplification thus conducted with use of the tagged mutation-introducing primer, can change the secondary structure of the target gene to be amplified so as to increase the proportion of single-strand formation, thereby increasing the amount of binding of the fluorescent-labeled probe, and improving sensitivity and accuracy of the measurement.

### Reference Signs List

101 DNA
102 fluorescent-labeled probe
103 fluorescent dye
104 quencher
201 forward primer
202 reverse primer
203 fluorescent-labeled probe
204 target DNA
205 molecule having complementary strands of target DNA in the form of double-stranded structure
206 molecule having fluorescent-labeled probe bound to single-stranded molecule of target DNA
207 one strand of target DNA
301 forward primer
302 mutation-introducing reverse primer
303 fluorescent-labeled probe
304 target DNA
305 secondary structure of single-stranded target DNA, amplified with non-mutated reverse primer
306 site planned for mutation introduction
307 secondary structure of single-stranded target DNA, amplified with mutation-introducing reverse primer
308 introduced mutation
309 mutation
401 forward primer
402 tagged mutation-introducing reverse primer
403 fluorescent-labeled probe
404 target DNA
405 tag sequence
406 secondary structure of single-stranded target DNA, amplified with non-mutated reverse primer
407 site planned for mutation introduction
408 secondary structure of single-stranded target DNA, amplified with tagged mutation-introducing reverse primer
409 introduced mutation
410 added tag sequence
411 mutation
501 melting temperature
701 secondary structure of single-stranded target DNA, amplified with non-mutated reverse primer
702 binding region for fluorescent-labeled probe adapted to wild-type
703 binding region for fluorescent-labeled probe adapted to mutant
704 peak regarding fluorescent-labeled probe adapted to amplified G13D mutant DNA and wild-type
705 peak regarding fluorescent-labeled probe adapted to amplified G13D mutant DNA and mutant
801 secondary structure of single-stranded G13D mutant DNA amplified without mutation-introducing primer
802 binding region for fluorescent-labeled probe
803 position planned for mutation introduction
804 secondary structure of single-stranded G13D mutant DNA amplified with use of mutation-introducing primer
805 position of mutation introduction
901 secondary structure of single-stranded G13D mutant DNA amplified without mutation-introducing primer
902 binding region for fluorescent-labeled probe
903 position planned for mutation introduction
904 secondary structure of single-stranded G13D mutant DNA amplified with use of mutation-introducing primer
905 position of mutation introduction
906 tag sequence

### [Sequence Listing Free Text]

SEQ ID Nos: 1-7: DNA (artificial sequence, synthetic polynucleotide)

## Claims

1. A method for detecting a target nucleic acid, the method comprising:
amplifying a test nucleic acid in the presence of a primer pair comprising a forward primer and a reverse primer, and a probe; and
measuring binding of a nucleic acid amplified by the forward primer and the reverse primer, with the probe,
wherein either the forward primer or the reverse primer, or both comprise, in the sequence, a mutation to be introduced into the amplified nucleic acid, so as to increase a proportion of single-strand forming bases in a probe binding region of the amplified nucleic acid, at a temperature of the binding of the amplified nucleic acid with the probe.

2. The method according to claim 1, wherein the reverse primer comprises the mutation.

3. The method according to claim 1, wherein the probe comprises a fluorescent dye, or comprises a fluorescent dye and a quenching dye, and the method comprises measuring binding of the amplified nucleic acid with the probe, with use of the fluorescent dye.

4. The method according to claim 1, wherein a 3' end sequence and a 5' end sequence of the probe are complementary.

5. The method according to claim 1, wherein the amplifying step is conducted by asymmetric nucleic acid amplification.

6. The method according to claim 1, wherein either the forward primer or the reverse primer, or both further comprise a tag sequence, so that the amplified nucleic acid will have the tag sequence comprised therein.

7. The method according to claim 1, wherein a proportion of the single-strand forming bases in the probe binding region of the amplified nucleic acid is 50% or larger.

8. The method according to claim 1, wherein the binding of the amplified nucleic acid with the probe is measured with changing temperature, and the method comprises estimating a melting temperature of a double strand of the amplified nucleic acid and the probe, from a change in the binding due to the temperature change.

9. The method according to claim 1, wherein binding of the amplified nucleic acid with the probe is measured for each cycle of temperature change, and the method comprises detecting the target nucleic acid, with reference to a relationship between the number of cycles and changes in the binding.

10. The method according to claim 1, comprising detecting a plurality of target nucleic acids, with use of a plurality of types of primer that comprises the mutation.

11. The method according to claim 10, wherein binding of the amplified nucleic acid with the probe is measured for each cycle of temperature change, and the method comprises estimating a proportion of initial concentrations of the plurality of target nucleic acids in the test nucleic acid, with reference to a relationship between the number of cycles and changes in the binding.

12. The method according to claim 1, wherein binding of the amplified nucleic acid with the probe is measured for each cycle of temperature change, and the method comprises comparing a relationship between the number of cycles and changes in the binding, with a change in the binding of a sample that comprises a known concentration of the target nucleic acid, and estimating an initial concentration of the target nucleic acid whose concentration is unknown in the test nucleic acid.

13. The method according to claim 1, wherein the detection of the target nucleic acid comprises determination of a genotype of the target nucleic acid.

14. A primer for introducing a mutation into a test nucleic acid and amplifying the test nucleic acid, the primer comprising, in its sequence, a mutation to be introduced into the test nucleic acid, so as to increase a proportion of single-strand forming bases in a probe binding region of the nucleic acid amplified from the test nucleic acid, at a temperature of the binding of the nucleic acid amplified from the test nucleic acid with the probe.

15. The primer according to claim 14, which is a reverse primer.

16. The primer according to claim 14, further comprising a tag sequence.

17. A kit for detecting a target nucleic acid, the kit comprising:
a primer pair that comprises a forward primer and a reverse primer; and
a probe that binds to a nucleic acid amplified with use of the forward primer and the reverse primer,
wherein either the forward primer or the reverse primer, or both comprise, in the sequence, a mutation to be introduced into the amplified nucleic acid, so as to increase a proportion of single-strand forming bases in a probe binding region of the amplified nucleic acid, at a temperature of the binding of the amplified nucleic acid with the probe.

18. The kit according to claim 17, wherein either the forward primer or the reverse primer, or both further comprise a tag sequence.

19. The kit according to claim 17, wherein concentration of the forward primer and concentration of the reverse primer are different.

20. The kit according to claim 17, wherein the probe comprises a fluorescent dye, or comprises a fluorescent dye and a quenching dye.

21. The kit according to claim 17, wherein a 3' end sequence and a 5' end sequence of the probe are complementary.

22. The kit according to claim 17, wherein a proportion of the single-strand forming bases in the probe binding region of the amplified nucleic acid is 50% or larger.

23. The kit according to claim 17, comprising a plurality of types of the primer pair comprising the forward primer and the reverse primer, individually adapted to a plurality of target nucleic acids, and/or comprising a plurality of types of the probe individually adapted to a plurality of target nucleic acids.

24. The kit according to claim 17, further comprising a DNA polymerase.
